(19) Europäisches Patentamt
European Patent Office
Office européen des brevets

(11) Veröffentlichungsnummer: **0 002 828**
**B1**

(12) # EUROPÄISCHE PATENTSCHRIFT

(45) Veröffentlichungstag der Patentschrift: **06.01.82**

(51) Int. Cl.³: **A 61 K 7/13** // C07C91/40

(21) Anmeldenummer: **78101849.4**

(22) Anmeldetag: **27.12.78**

(54) **Haarfärbemittel.**

(30) Priorität: **27.12.77 DE 2758203**

(43) Veröffentlichungstag der Anmeldung: **11.07.79 Patentblatt 79/14**

(45) Bekanntmachung des Hinweises auf die Patenterteilung: **06.01.82 Patentblatt 82/1**

(84) Benannte Vertragsstaaten: **BE CH FR GB NL SE**

(56) Entgegenhaltungen:
**CH-A-455 154**
**FR-A-1 136 996**
**GB-A-822 948**
**GB-A-1 287 343**
**US-A-2 083 308**
**CHEMICAL ABSTRACTS, Vol. 75, Nr. 20, 15. November 1971, Columbus, Ohio, USA, T. KINOSHITA »Compositions for dieing hair«, Seite 210, Spalte 1, Zusammenfassung Nr. 121294f**

(73) Patentinhaber: **Henkel Kommanditgesellschaft auf Aktien, -Patentabteilung- Postfach 1100 Henkelstrasse 67, D-4000 Düsseldorf 1 (DE)**

(72) Erfinder: **Rose, David, Dr., Holbeinweg 7, D-4010 Hilden (DE)**
Erfinder: **Lieske, Edgar, Hunsrückenstrasse 40, D-4000 Düsseldorf (DE)**
Erfinder: **Busch, Peter, Dr., Gottfried-August-Bürger-Strasse 10, D-4006 Erkrath-1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Haarfärbemittel

Gegenstand der Erfindung sind Mittel zur oxidativen Färbung von Haaren auf Basis von N,N-Bis-($\beta$-hydroxyäthyl)-m-phenylendiaminen als Kupplerkomponente.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen, wie p-Phenylendiaminderivate, Diaminopyridine, 4-Aminopyrazolonderivate, heterocyclische Hydrazone eingesetzt. Als sogenannte Kupplerkomponenten werden Phenole, Naphthole, Resorcinderivate und Pyrazolone genannt.

Gute Oxidationshaarfarbstoffkomponenten müssen in erster Linie folgende Voraussetzungen erfüllen:

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen, und sie sollen darüber hinaus in toxikologischer und dermatologischer Hinsicht unbedenklich sein. Weiterhin ist von Bedeutung, daß auf dem zu färbenden Haar möglichst kräftige und den natürlichen Haarfarbnuancen weitgehend entsprechende Farbtöne erhalten werden. Ferner kommt der allgemeinen Stabilität der gebildeten Farbstoffe sowie deren Lichtechtheit, Waschechtheit und Thermostabilität ganz besondere Bedeutung zu, um Farbverschiebungen von der ursprünglichen Farbnuance oder gar Farbumschläge in andere Farbtöne zu vermeiden.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurde nun gefunden, daß Haarfärbemittel auf Basis von Oxidationsfarbstoffen mit einem Gehalt an N,N-Bis-($\beta$-hydroxyäthyl)-m-phenylendiaminen der allgemeinen Formel

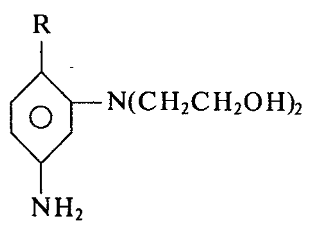

in der R Wasserstoff oder eine Alkoxy-Gruppe, deren Alkylrest 1–4 C-Atome enthält, darstellt, sowie deren anorganischen oder organischen Salzen als Kupplersubstanzen und den in Oxidationshaarfarben üblichen Entwicklerkomponenten den gestellten Anforderungen in besonders hohem Maße gerecht werden.

Bei ihrem Einsatz als Kupplerkomponenten liefern die erfindungsgemäßen Verbindungen mit den im allgemeinen für die Oxidationshaarfärbung verwendeten Entwicklersubstanzen sehr intensive von gelbbraun bis violett reichende Farbtöne und stellen somit eine wesentliche Bereicherung der oxidativen Haarfärbemöglichkeiten dar. Darüber hinaus zeichnen sich die erfindungsgemäßen N,N-Bis-($\beta$-hydroxyäthyl)-m-phenylendiamine durch sehr gute Echtheitseigenschaften der damit erzielten Färbungen, durch eine gute Löslichkeit in Wasser, eine gute Lagerstabilität und toxikologische sowie dermatologische Unbedenklichkeit aus.

Die erfindungsgemäß als Kupplerkomponenten zu verwendenden N,N-Bis-($\beta$-hydroxyäthyl)-m-phenylendiamine können entweder als solche oder in Form ihrer Salze mit anorganischen oder organischen Säuren, wie z. B. als Chloride, Sulfate, Phosphate, Acetate, Propionate, Lactate, Citrate eingesetzt werden.

Die erfindungsgemäß als Kupplerkomponenten zu verwendenden N,N-Bis-($\beta$-hydroxyäthyl)-m-phenylendiamine stellen literaturbekannte Verbindungen dar, deren Herstellung zum Beispiel durch Säureabspaltung aus den entsprechenden handelsüblichen N-Acetylverbindungen erfolgen kann.

Als erfindungsgemäß zu verwendende Kupplerkomponenten sind N,N-Bis-($\beta$-hydroxyäthyl)-m-phenylendiamin und 2-Methoxy-, 2-Äthoxy-, 2-Propoxy-, 2-Butoxy-5-amino-N,N-bis-($\beta$-hydroxyäthyl)-anilin zu nennen.

Als Beispiel für in den erfindungsgemäßen Haarfärbemitteln einzusetzende Entwicklerkomponenten sind primäre aromatische Amine mit einer weiteren in p-Stellung befindlichen funktionellen Gruppe wie p-Phenylendiamin, p-Toluylendiamin, p-Aminophenol, N-Methyl-p-phenylendiamin, N,N-Dimethyl-p-phenylendiamin, N,N-Diäthyl-2-mehtyl-p-phenylendiamin, N-Äthyl-N-hydroxyäthyl-p-phenylendiamin, Chlor-p-phenylendiamin, N,N-Bis-hydroxyäthylamino-p-phenylendiamin, Methoxy-p-phenylendiamin, 2,6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1–4 Kohlenstoffatomen darstellt, ferner

Diaminopyridinderivate, heterocyclische Hydrazonderivate wie 1-Methyl-pyrrolidon-(2)-hydrazon, 4-Aminopyrazolonderivate wie 4-Amino-1-phenyl-3-carbamoylpyrazolon-5, N-Butyl-N-sulfobutyl-p-phenylendiamin, Tetraaminopyrimidine wie 2,4,5,6-Tetraaminopyrimidin, 4,5-Diamino-2,6-bis-methylaminopyrimidin, 2,5-Diamino-4-diäthylamino-6-methylaminopyrimidin, 2,4,5-Triamino-6-dimethylaminopyrimidin, 2,4,5-Triamino-6-piperidino-pyrimidin, 2,4,5-Triamino-6-anilino-pyrimidin, 2,4,5-Triamino-6-morpholino-pyrimidin, 2,4,5-Triamino-6-$\beta$-hydroxyäthylamino-pyrimidin anzuführen.

Zur Erzielung möglichst kräftiger und den natürlichen Haarfarbennuancen weitgehend entsprechender Farbtöne ist ein vollwertiger Blaufarbstoff als Nuancierkomponente von besonderer Wichtigkeit. Bei der Erstellung natürlicher Farbnuancen unter Zuhilfenahme von Blaukupplerkomponenten ergeben sich mit den üblichen Blaukupplern häufig Schwierigkeiten. Diesem Mangel kann durch den Einsatz der erfindungsgemäß zu verwendenden N,N-Bis-($\beta$-hydroxyäthyl)-m-phenylendiamine weitgehend abgeholfen werden. Diese Kupplerkomponenten liefern mit entsprechenden Entwicklersubstanzen neben anderen Farbnuancen dunkelblaue bis schwarzblaue, besonders intensive Haarfärbungen, die sich durch außerordentliche Lichtechtheiten auszeichnen.

In den erfindungsgemäßen Haarfärbemitteln werden die Kupplerkomponenten im allgemeinen in etwa molaren Mengen, bezogen auf die verwendeten Entwicklersubstanzen, eingesetzt. Wenn sich auch der molare Einsatz als zweckmäßig erweist, so ist es jedoch nicht nachteilig, wenn die Kupplerkomponente in einem gewissen Überschuß oder Unterschuß zum Einsatz gelangt.

Es ist ferner nicht erforderlich, daß die Entwicklerkomponente und die Kupplersubstanz einheitliche Produkte darstellen, vielmehr können sowohl die Entwicklerkomponente Gemische der erfindungsgemäß zu verwendenden Entwicklerverbindungen als auch die Kupplersubstanz Gemische der erfindungsgemäß einzusetzenden N,N-Bis-($\beta$-hydroxyäthyl)-m-phenylendiamine darstellen.

Darüber hinaus können die erfindungsgemäßen Haarfärbemittel gegebenenfalls übliche direktziehende Farbstoffe im Gemisch enthalten, falls dies zur Erzielung gewisser Farbnuancen erforderlich ist.

Die oxidative Kupplung, d. h. die Entwicklung der Färbung, kann grundsätzlich wie bei anderen Oxidationshaarfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Die erfindungsgemäßen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwickler-Kombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind z. B. Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Äthylenoxid an Fettalkohole, Verdickungsmittel wie Methylcellulose, Stärke, höhere Fettalkohole, Paraffinöl, Fettsäuren, ferner Parfümöle und Haarpflegemittel wie Pantothensäure und Cholesterin zu nennen. Die genannten Zusatzstoffe werden dabei in den für diese Zwecke üblichen Mengen eingesetzt, wie z. B. Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8 bis 10 erfolgen. Die Anwendungstemperaturen bewegen sich dabei im Bereich von 15 bis 40°C. Nach einer Einwirkungsdauer von ca. 30 Minuten wird das Haarfärbemittel vom zu färbenden Haar durch Spülen entfernt.

Hiernach wird das Haar mit einem milden Shampoo nachgewaschen und getrocknet.

Aus der britischen Patentschrift 1 287 343 sind bereits Haarfärbemittel bekannt, die als Farbstoffkomponenten m-Phenylendiamin-Derivate enthalten, in denen die eine Aminogruppe durch Carbamoylethylreste substituiert ist. Durch die Carbonamidgruppe wird die Eignung derartiger Produkte als Haarfärbemittel in ungünstiger Weise beeinflußt, so ist die Reibechtheit des gefärbten Substrats nicht befriedigend und die Lagerstabilität der mit ihnen hergestellten Färbecremes ist ungenügend. Ferner beschreibt die Schweizerische Patentschrift 455 154 wäßrige Färbezusammensetzungen zum Färben menschlicher Haare auf Basis von Nitroanilinen, die in der Aminogruppe durch Hydroxyethylreste substituiert sind. Hierbei handelt es sich um Verbindungen, die sowohl von ihrer Struktur als auch von ihrem Charakter als direktziehende Farbstoffe her wenig Beziehung zu den erfindungsgemäß zu verwendenden Kupplerkomponenten haben.

Die nachfolgenden Beispiele sollen den Erfindungsgenstand näher erläutern, ohne ihn jedoch hierauf zu beschränken.

## Beispiele

Als Kupplersubstanzen wurden in den folgenden Beispielen die nachfolgend genannten Verbindungen eingesetzt:

A: N,N-Bis-(β-hydroxyäthyl)-m-phenylendiamin,
B: 2-Methoxy-5-amino-N,N-bis-(β-hydroxyäthyl)-anilin,
C: 2-Äthoxy-5-amino-N,N-bis-(β-hydroxyäthyl)-anilin.

Als Entwicklerkomponenten dienten folgende Substanzen:

E1: p-Phenylendiamin
E2: p-Toluylendiamin
E3: p-Aminophenol
E4: N,N-Dimethyl-p-phenylendiamin
E5: 2-Piperidino-4,5,6-triaminopyrimidin
E6: 1-Methyl-pyrrolidon-(2)-hydrazon
E7: 1-Phenyl-3-carbamoyl-4-aminopyrazolon
E8: N,N-Bis-(β-hydroxyäthylamino)-p-phenylendiamin
E9: N-Butyl-N-sulfobutyl-p-phenylendiamin
E10: 2,4,5,6-Tetraaminopyrimidin
E11: 2-Chlor-1,4-diaminobenzol

Die erfindungsgemäßen Haarfärbemittel wurden in Form einer Cremeemulsion eingesetzt. Dabei wurden in eine Emulsion aus

10 Gew.-Teilen Fettalkoholen der Kettenlänge $C_{12}-C_{18}$
10 Gew.-Teilen Fettalkoholsulfat (Natriumsalz) Kettenlänge $C_{12}-C_{18}$
75 Gew.-Teilen Wasser

jeweils 0,01 Mol der in der nachstehenden Tabelle aufgeführten Entwicklersubstanzen und N,N-Bis-(β-hydroxyäthyl)-m-phenylendiamine eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde entweder mit Luftsauerstoff oder mit 1%iger Wasserstoffperoxidlösung als Oxidationsmittel durchgeführt, wobei zu 100 Gewichtsteilen der Emulsion 10 Gewichtsteile Wasserstoffperoxidlösung gegeben wurden. Die jeweilige Färbecreme mit oder ohne zusätzlichem Oxidationsmittel wurde auf zu 90% ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

Tabelle 1

| Beispiel | a) Entwickler | b) Kuppler | Erhaltener Farbton bei Luftoxidation | mit 1%iger $H_2O_2$-Lösung |
|---|---|---|---|---|
| 1) | E 1 | A | dunkelblau | dunkelblau |
| 2) | E 2 | A | dunkelblau | dunkelblau |
| 3) | E 3 | A | graurubin | graumagenta |
| 4) | E 4 | A | dunkelblau | dunkelblau |
| 5) | E 5 | A | braun | rotbraun |
| 6) | E 6 | A | gelbbraun | braunorange |
| 7) | E 7 | A | dunkelblau | dunkelblau |
| 8) | E 8 | A | dunkelblau | dunkelblau |
| 9) | E 9 | A | grautürkis | dunkelblau |
| 10) | E 10 | A | olivbraun | olivbraun |
| 11) | E 1 | B | schwarzblau | schwarzblau |
| 12) | E 3 | B | violettbraun | rotbraun |
| 13) | E 4 | B | dunkelblau | dunkelblau |
| 14) | E 5 | B | mattgrün | mattgrün |
| 15) | E 6 | B | gelbbraun | braunorange |
| 16) | E 7 | B | blaugrau | blaugrau |
| 17) | E 1C | B | dunkelgrün | grün |
| 18) | E 8 | B | dunkelblau | dunkelblau |
| 19) | E 9 | B | mattblau | mattblau |
| 20) | E 11 | B | grauviolett | mattviolett |
| 21) | E 1 | C | dunkelblau | dunkelblau |
| 22) | E 2 | C | dunkelblau | dunkelblau |

**Patentansprüche**

1. Haarfärbemittel auf Basis von Oxidationsfarbstoffen, gekennzeichnet durch einen Gehalt an N,N-Bis-(β-hydroxyäthyl)-m-phenylendiaminen der allgemeinen Formel

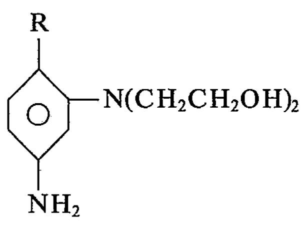

in der R Wasserstoff oder eine Alkoxy-Gruppe, deren Alkylrest 1 – 4 C-Atome enthält, darstellt, sowie deren anorganischen oder organischen Salzen als Kupplersubstanzen und den in Oxidationsfarben üblichen Entwicklerkomponenten.

2. Haarfärbemittel nach Anspruch 1, gekennzeichnet durch einen Gehalt weiterer üblicher Kupplersubstanzen sowie gegebenenfalls üblicher direktziehender Farbstoffe.

3. Haarfärbemittel nach Anspruch 1 und 2, gekennzeichnet durch einen Gehalt an Entwickler-Kuppler-Kombinationen von 0,2 bis 5 Gewichtsprozent, vorzugsweise von 1 – 3 Gewichtsprozent.

## Claims

1. A hair tinting agent based on oxidation dyes, characterised in that it contains N,N-bis-(β-hydroxyethyl)-m-phenylene diamines corresponding to the following general formula

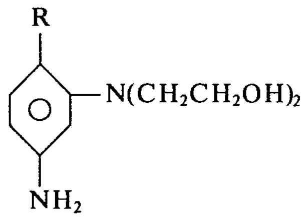

in which R represents hydrogen or an alkoxy group of which the alkyl radical contains from 1 to 4 carbon atoms, and their inorganic or organic salts as couplers and the developer components normally used for oxidation dyes.

2. A hair tinting agent as claimed in claim 1, characterised in that it contains other standard couplers and optionally standard substantive dyes.

3. A hair tinting agent as claimed in claims 1 and 2, characterised in that it contains from 0.2 to 5% by weight and preferably from 1 to 3% by weight of developercoupler combinations.

## Revendications

1. Agent de teinture de cheveux à base de colorants d'oxydation, caractérisé par une teneur en N,N-bis-(β-hydroxyéthyl)-m-phénylène diamines de formule générale:

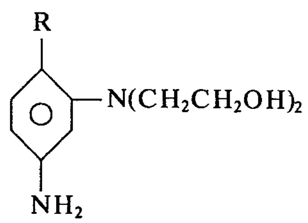

dans laquelle R représente de l'hydrogène ou un groupe alcoxy dont le radical alcoyle contient 1 à 4 atomes de carbone, ainsi que leurs sels minéraux ou organiques en tant que substances de couplage, et en composants de développement usuels dans les colorants d'oxydation.

2. Agent de teinture de cheveux selon la revendication 1, caractérisé par une teneur en d'autres substances usuelles ce couplage de même qu'éventuellement en d'autres colorants montants directs en usage courant.

3. Agent de teinture de cheveux selon les revendications 1 et 2, caractérisé par une teneur en combinaisons agent de développement-coupleur de 0,2 à 5% en poids, de préférence de 1 à 3% en poids.